# EUROPEAN PATENT APPLICATION

(11) **EP 1 288 303 A1**
(43) Date of publication of application: **05.03.2003**
(21) Application number: 01120270.2
(22) Date of filing: 23.08.2001
(51) Int. Cl.: C12N 15/85, C07K 14/705, C12N 15/67, C12Q 1/68

(54) **Inducible expression systems employing PPAR transcriptional activators**

(71) Applicant: Aventis Pharma S.A., 92160 Antony (FR)
(72) Inventor: Darteil, Raphael, 75011 Paris (FR)
(74) Representative: Lecca, Patricia

(57) **Abstract**

The invention relates to gene expression systems that employ novel variants of mammalian peroxisome proliferator-activated receptor (PPAR) proteins as transcriptional activators, as well as novel PPAR polypeptides and nucleic acids that encode them.

## Description

The invention relates to gene expression systems that employ novel variants of mammalian peroxisome proliferator-activated receptor (PPAR) proteins as transcriptional activators, as well as novel PPAR polypeptides and nucleic acids that encode them. The transcriptional activator, the improvement in transcriptional control and dose-response characteristics, and the compounds used for inducing expression in the systems and methods of the invention each provide advantages over other available systems.

Inducible expression systems used in gene transfer vectors suffer from two main deficiencies: expression levels cannot be controlled adequately to ensure a substantial lack of transcription in certain cellular environments; and they require the use of proteins or compounds that effect the cell or organism in other, inappropriate or undesirable ways. For example, many inducible expression systems employ heterologous or chimeric proteins, which are immunogenic in animals. Other systems allow unacceptably high levels of background expression in a cell. This invention addresses these and other shortcomings in the art by providing a variety of expression systems employing novel PPAR polypeptides, transcriptional activators, and the nucleic acids that encode them. Furthermore, the range of induction compounds possible results in the ability to design systems with superior side effect profiles compared to other systems. Induction compounds that complement the intended action of the induced gene can also be selected, depending on the application intended.

The mechanisms for controlling when gene expression is turned on and off have been an important aspect of biological research for decades. The earliest work in bacteriophage operons indicated that certain compounds effect when a gene is turned on to produce a protein product. These compounds are said to induce transcription or expression.

As the ability to manipulate cells and organisms to produce different proteins advanced, so to has the ability to control when protein production is turned on. In some circumstances, control is crucial. For example, some proteins possess cytopathic properties that kill the cells they are produced in. If no reliable mechanism controls the production of these cytopathic proteins, it would be difficult if not impossible to produce the proteins in a recombinant expression system.

The use of compounds to induce or turn on gene expression allows a degree of control over when a protein is produced. Expression systems incorporating nucleic acid sequences that respond only to the presence of these compounds were constructed. These expression systems have been called inducible expression systems. Applying these systems to the development of gene transfer vectors for animals and the treatment of disease in animals, however, implicates a number of safety and reliability concerns. Most of these concerns did not exist or were not relevant to the development of vectors containing inducible expression systems.

A few inducible expression systems have been demonstrated in animal cells using gene transfer vectors. In fact, inducible expression systems can be used in a variety of plasmid and viral vectors. Much of the attention has involved the tetracycline regulatable system or the ecdysone regulatable system, systems induced by the drug tetracycline or a ligand of the ecdysone receptor. For example, Clontech (Palo Alto, CA) produces a tetracycline-regulatable adenovirus vector system for transient gene expression. Stratagene (La Jolla, CA) produces a retroviral expression system employing ecdysone receptors. And Ariad (Cambridge, MA) produces a series of regulatable vectors, such as a plasmid kit, that use a chimeric FKBP (FK-506 binding protein) and analogs of the immunosuppressive drug rapamycin. Some of these examples are being used in human clinical applications for treating disease.

These systems suffer from the very real prospect of or reality that they create deleterious side effects in the animals they are used with. For example, chimeric proteins and other foreign proteins produce immune responses when introduced into an animal, a response that could pose serious complications if long-term use is intended. Thus, expression systems that employ foreign proteins will have to address an undesired immune response. Furthermore, the compounds or drugs used to induce expression may have unwanted effects on the animal, for example, immunosuppression. Systems that show the lowest amount of expression when they are supposed to be off, or when no compounds to induce expression are present, will generally be preferred over other systems. For these and other reasons, researchers in the art continue to seek improved, inducible expression systems for animal and mammalian gene transfer and gene therapy vectors. The present invention, in some aspects, satisfies this need. In other aspects, the invention provides novel nucleic acids and polypeptides for use in a variety of biological applications. Additional aspects are described below.

The invention encompasses new PPAR polypeptides and nucleic acids encoding them. The PPAR polypeptides and nucleic acids can be used in inducible mammalian expression systems, which systems greatly increase the specificity and the safety of gene transfer-based expression. In one aspect, the new PPAR polypeptides reduce the risk of side effects as their use does not activate endogenous genes in the cells or animals subjected to gene transfer. For example, PPARγ is involved in adipocyte differentiation, the process of inducing the maturation of pre-adipocytes into mature fat cells. Overexpression of wild type PPARγ can lead to adipocyte differentiation. The novel PPAR polypeptides of the invention, when overexpressed in cells, do not cause differentiation. In addition, they can be used in human cells and tissue without triggering an adverse immune response. Other regulatable expression systems can cause immune responses (Latta-Mahieu, *et al.,* Molecular Therapy, vol.3, number 5, part 2, May 2001, abstract 1133).

The invention also encompasses new response elements that can be used in conjunction with PPAR-based transcriptional transactivators, such as with an inducible expression system. The response elements comprise nucleic acids. The nucleic acids have nucleotide sequences that allow the binding of a PPAR heterodimer, which operates to control transcription levels in the presence or absence of specific ligands for PPAR. Accordingly, the response elements can be incorporated into an expression vector by operably linking the response element to the promoter in the vector. In this way, the PPAR transcriptional activator will control the expression of the gene linked to the promoter depending on whether or not a specific ligand for PPAR is present.

In an important aspect, the invention comprises inducible expression systems and methods for using the inducible expression systems to control the expression of a gene of interest in a mammalian cell or animal. The expression systems comprise one or more of the new PPAR polypeptides and one or more of the response elements noted above. Typically, the expression systems comprise a nucleic acid that encodes the new PPAR polypeptides linked to a promoter or transcription control sequences. The systems also comprise a nucleic acid comprising a promoter sequence operably linked to a response element and a gene of interest, so that the binding of a PPAR transcriptional activator control the expression of the gene of interest. Any of a variety of types of genes can be selected as the gene of interest. The nucleic acids noted above can be present on a single vector for introduction into a cell or animal, or they can be present on separate vectors.

The methods of using the inducible expression systems and can be incorporated into a treatment for various diseases or conditions based on a gene transfer protocol. In these embodiments, a gene of interest expressed from the inducible expression system can be any therapeutic protein, such as any of those capable of being used or being used in clinical trials, or any therapeutic transcript, such a functional RNA or anti-sense sequences. One skilled in the art is familiar with numerous ways to identify and use therapeutic proteins, fragments of them, and therapeutic transcripts or transgenes. Alternatively, the expression systems can be used to identify or screen for therapeutic genes or treatment regimens. One skilled in the art is familiar with numerous ways to use the components of inducible expression systems and vectors.

In particular embodiments, any aspect of the invention may comprise a PPAR polypeptide comprising a Zinc finger amino acid domain that comprises the amino acid sequence selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV], or comprising SEQ ID No: 8 [PPARγ2 (GSV)] or SEQ ID No: 7 [PPARγ2 (GSG)]. In particular embodiments, any aspect of the invention may comprise a response element comprising the nucleotides of SEQ ID Nos: 10-40, especially 22, 24, 26, 28, 34, and 36, or any of SEQ ID Nos.: 10, 12, 14, 16, 18, 20, 22, 24, 26, 28, 30, 32, 34, 36, 38, and 40. However, additional PPAR polypeptides and response element sequences can be produced and selected for use according to the invention. Thus, the specific sequences listed here should not be taken as a limitation on the scope of the invention. A number of different possible PPAR ligands can also be incorporated into aspects of the invention. One skilled in the art is familiar with the PPAR ligands and with the methods to screen them for the ability to control gene expression, as shown here.

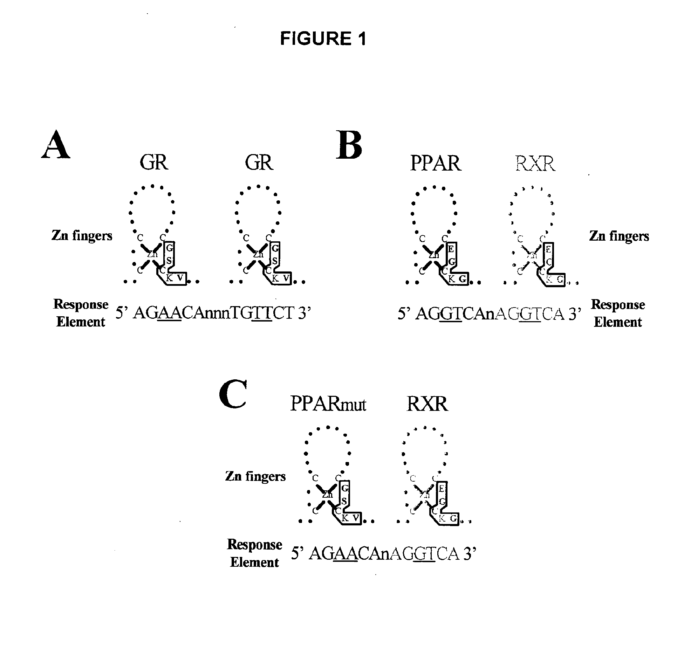

In more general terms, the PPAR-based inducible expression system of the invention is a new gene switch system based on human Peroxisome Proliferator Activated Receptors (PPAR). PPARs are members of the nuclear hormone superfamily, which have common structural features. They generally contain an N-terminal domain, a DNA-binding domain (DBD), and a ligand-binding domain (LBD) at the C-terminus. The PPARs form heterodimers with another nuclear receptor, the 9-cis-retinoic acid receptor, RXR (retinoid X receptor). Once formed, the PPAR/RXR heterodimers bind to DNA sequences containing direct repeats of the hexanucleotide sequence AGGTCA separated by one nucleotide ("n" = any nucleotide, preferably A), known as DR-1 response elements (Figure 1B). This sequence, also called the PPAR Response Element (PPRE), has been characterized within the promoter region of PPAR target genes, or genes that are regulatable by PPAR/RXR heterodimer binding. This led to the discovery that the 5 base pairs immediately upstream of PPAR core binding (DR-1) site are important modulators of the binding affinity of the PPAR/RXR heterodimer to the PPRE. As discussed here, the response elements of the invention may include these 5 additional bases pairs. While the invention is not limited by or bound by any particular explanation or theory on how the expression system works in the cell, we believe that when a PPAR ligand binds to the heterodimer, a recruitment of coactivator proteins within the cell leads to transcriptional activation of the target gene, which is linked to the response element. In this manner and using the knowledge presented here, one skilled in the art can construct many variations in the selection of any of the PPAR polypeptide, response element, and PPAR ligand to achieve inducible and highly regulatable expression of a gene of interest in any particular cell selected. Preferably, the cells and animals selected are mouse, primate, or human and even more preferably myocytes (muscle cells) or adipocytes (fat cells). Furthermore, the invention encompasses methods for identifying or optimizing the selection of one element of the expression system, the response element for example, after selecting a PPAR polypeptide of the invention and suitable ligand. The method involves selecting a nucleic acid encoding a PPAR polypeptide comprising an amino acid sequence selected from SEQ ID No.: 1-6, inserting the nucleic acid into an expression vector, preparing an expression cassette on the same or a separate vector that comprises a response element operably linked to a minimal promoter or other promoter and a gene of interest, and testing for the expression levels of the gene of interest after introducing a PPAR ligand that binds to the selected PPAR polypeptide. Similarly, a selected response element can be chosen and a number of PPAR polypeptides tested for their ability to control inducible expression of the gene of interest. Each of or any combination of the specific PPAR polypeptides or P-box amino acid sequences listed throughout, or the response elements listed throughout, can be used in these methods.

### Brief Description of the Drawings

Figure 1: **Construction of PPAR polypeptide with unique DNA binding properties.** Schematic representation of the first Zinc finger domain of different nuclear receptors with their corresponding response elements. The P-box of the Zinc finger domain, which includes amino acids responsible for the specificity of target gene expression, is boxed. (**A**) Glucocorticoid Receptor (GR) homodimer and Glucocorticoid Response Element (GRE). (**B**) Wild type PPAR/RXR (retinoic acid receptor) heterodimer and wild type PPAR Response Element (PPRE). (**C**) PPAR polypeptide of the invention (PPARmut)/RXR heterodimer and response element.

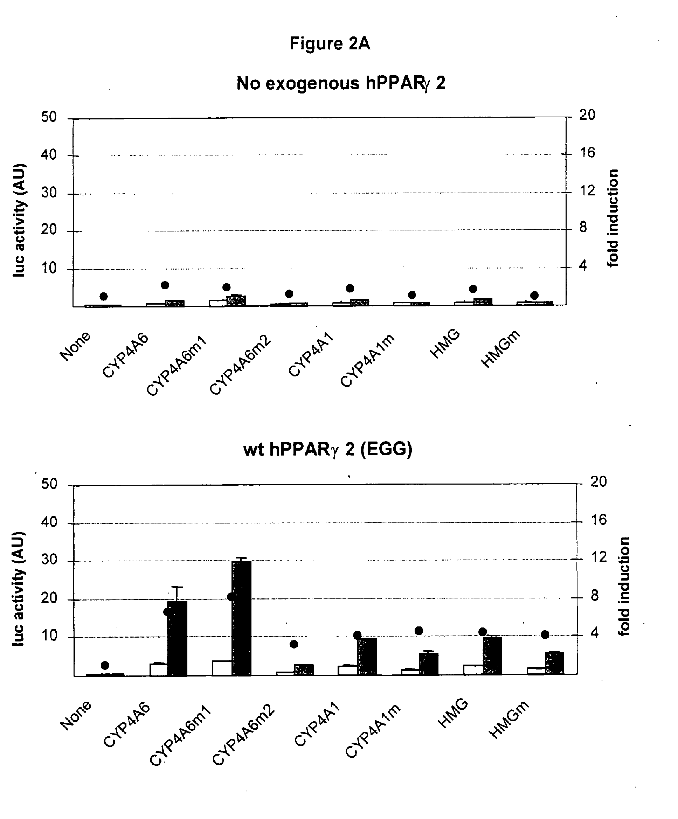

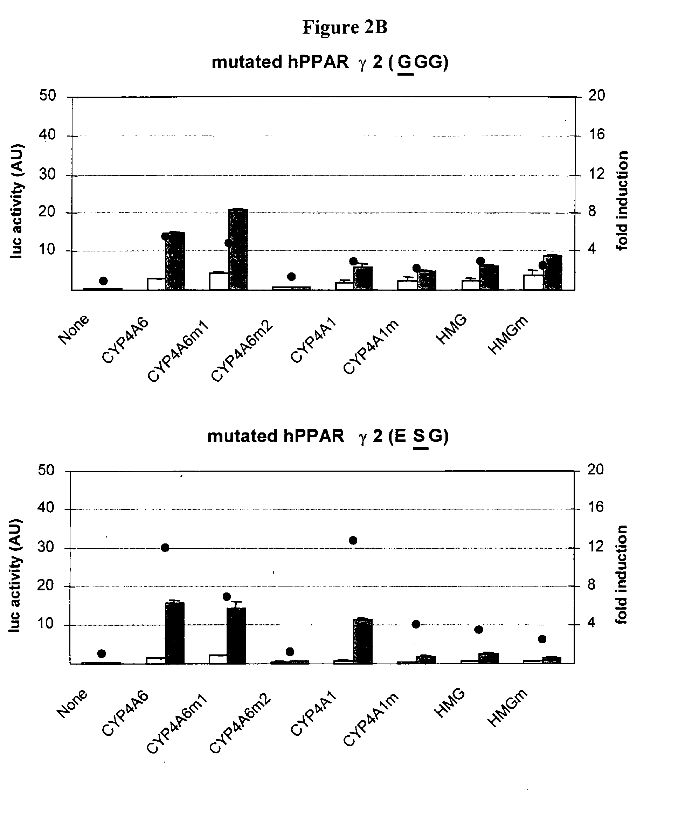

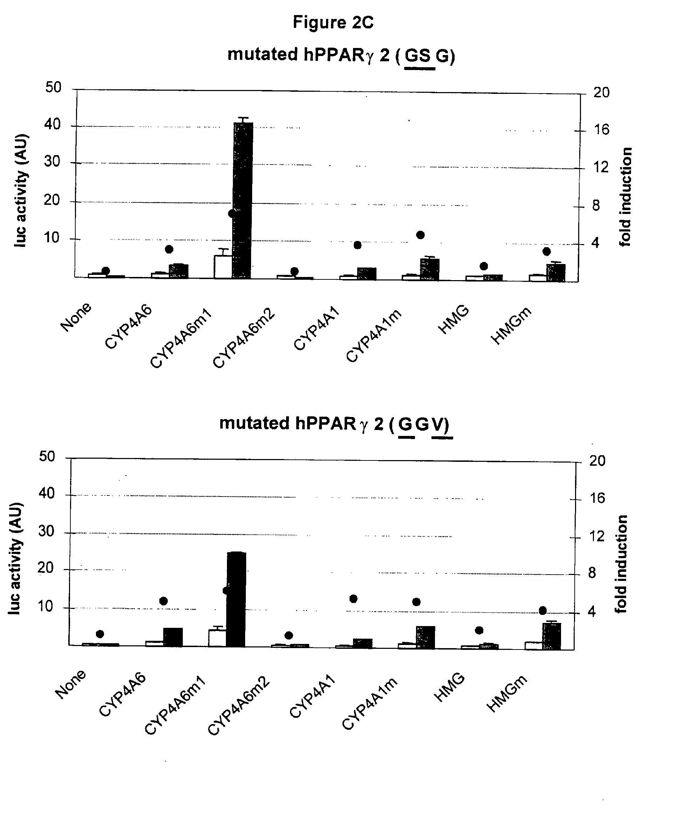

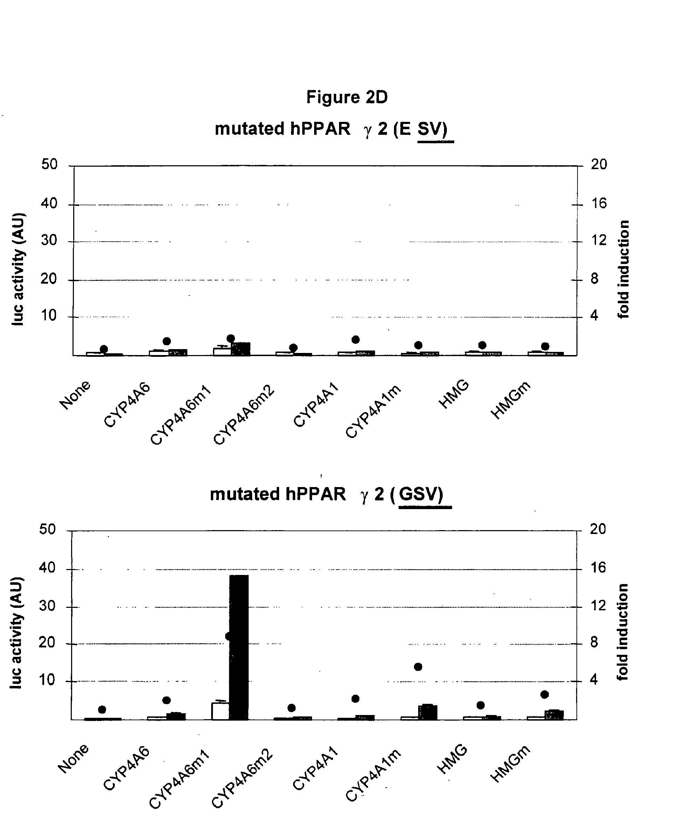

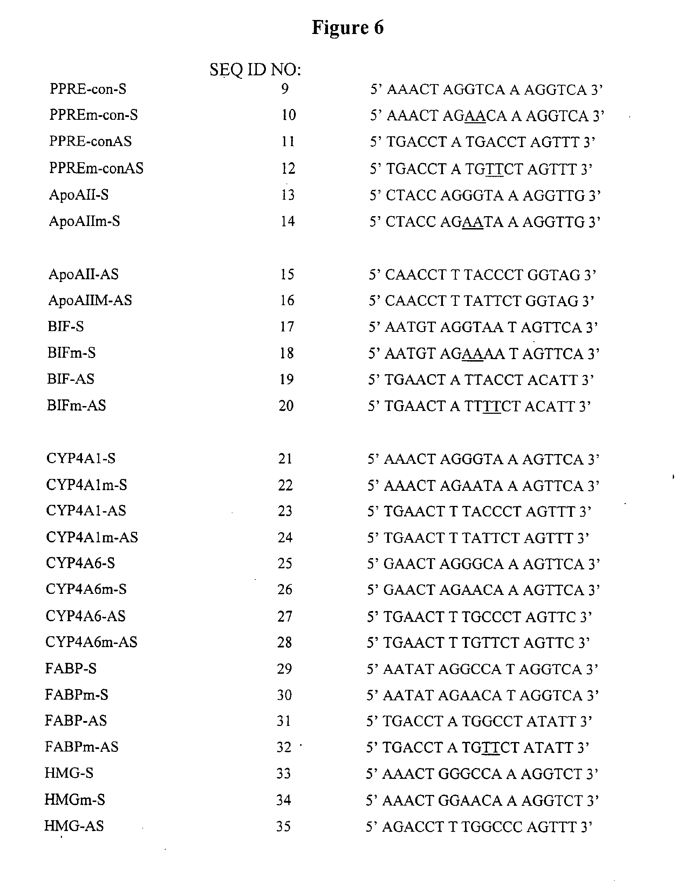

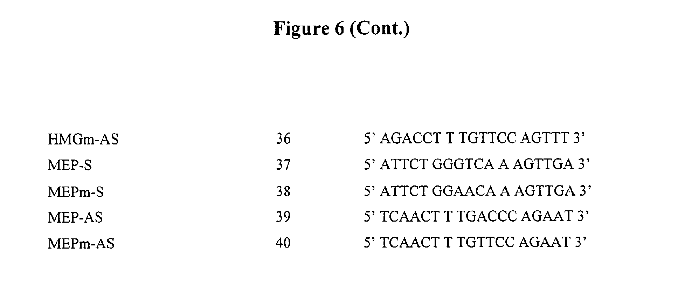

Figure 2: **Comparison of PPAR polypeptides derived from human PPARγ2 receptor.** C2C12 murine myoblasts were cotransfected with a mixture of 3 different plasmids : (i) a plasmid expressing the wt or a mutated hPPARγ2; (ii) a plasmid in which the luc+ gene expression is driven by an inducible target sequence made of multiple PPAR Response Elements (PPREs) of wt origin (CYP4A6, CYP4A1 and HMG) or mutated response sequence (CYP4A6m1 = SEQ ID No.: 26, CYP4A6m2 = control with sequence 5' GAACT AGGGCA A AGAACA 3' (SEQ ID No.: 41), CYP4A1m = SEQ ID No.: 22, and HMGm = SEQ ID No.: 34); and (iii) a plasmid expressing the *Renilla* luciferase gene as an internal control of transfection efficiency. Measurement of luciferase activity for untreated cells (white bars) or cells treated with the BRL49653 PPARγ ligand (grey bars) is shown for each response element (left Y-axis). The relative induction level (x fold induction) resulting from ligand treatment is also shown (dots and right Y-axis)). Nomenclature for the PPAR amino acid sequences used refers to the first, second, and fifth (bolded) amino acid in the P-box of the Zinc finger domain, where wild type is **EGCKG** (see box in Figure 1, panel B). The nucleotide sequence content of exemplary response elements, and those used here, is shown in Figure 6.
Figure 2A: Results for experiments in which plasmid (i) contains control (top) or wild type human PPARγ2 (bottom) PPAR receptor sequence with each response element bearing plasmid (ii) are shown. The "EGG" sequence refers to the first, second, and fifth amino acid in the P-box of the Zinc finger domain **EGCKG** (see box in Figure 1, panel B). Activity from both of the CYP4A6 (wt) and CYP4A6m1 (mutant) response elements is shown with the wild type PPARγ2 (bottom).
Figure 2B: Results for experiments in which plasmid (i) contains PPAR polypeptide with GGG amino acids at first, second, and fifth position of P-box (top) or with ESG at first, second, and fifth position of P-box (bottom) with each response element bearing plasmid (ii) are shown. Again, activity from both of the CYP4A6 (wt) and CYP4A6m1 (mutant) response elements is highest with both PPAR polypeptide.
Figure 2C: Results for experiments in which plasmid (i) contains PPAR polypeptide with GSG amino acids at first, second, and fifth position of P-box (top) or with GGV at first, second, and fifth position of P-box (bottom) with each response element bearing plasmid (ii) are shown. The activity from CYP4A6m1 (mutant) response elements is particularly strong and selective in both cases.
Figure 2D: Results for experiments in which plasmid (i) contains PPAR polypeptide with ESV amino acids at first, second, and fifth position of P-box (top) or with GSV at first, second, and fifth position of P-box (bottom) with each response element bearing plasmid (ii) are shown. The ESV receptor shows no activity. The activity from CYP4A6m1 (mutant) response elements combined with the GSV mutant is highly selective, specificity only for the CYP4A6m1 response element, and shows the highest induction levels.

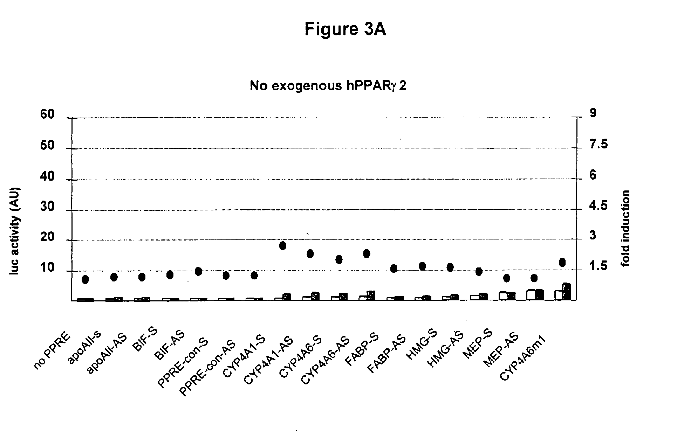

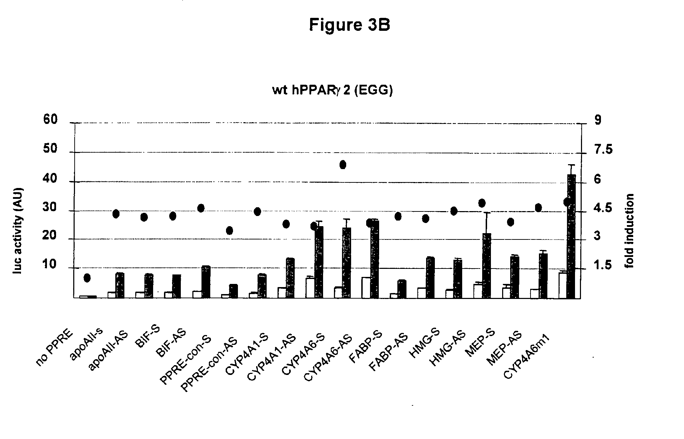

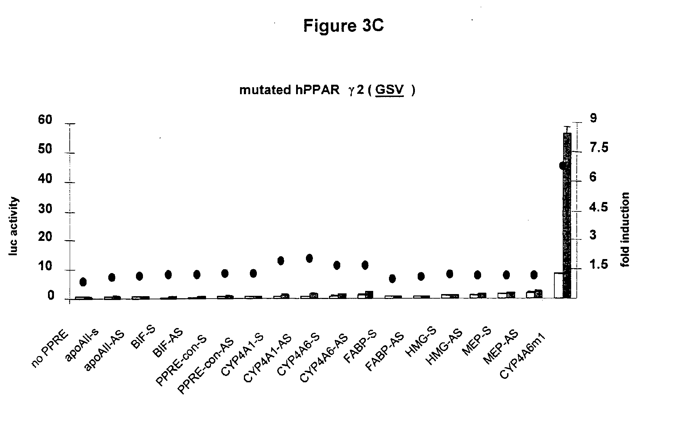

Figure 3: **DNA-binding specificity of the GSV mutant.** C2C12 murine myoblasts were cotransfected with a mixture of 3 different plasmids: (i) a plasmid expressing the wt EGG or the GSV mutated hPPARγ2; (ii) a plasmid in which the luc+ gene expression is driven by an inducible target sequence made of multiple wt PPREs (apoAII, BIF, PPREcon, CYP4A1, CYP4A6, FABP, HMG and MEP cloned in sense (-S) or anti-sense (-AS) orientation) or the mutated response element (noted as "m" sequences in Figure 6, such as CYP4A6m1 = SEQ ID No.: 26); and (iii) a plasmid expressing the *Renilla* luciferase gene as an internal control of transfection efficiency. Expression levels for untreated cells (white) or cells treated with the BRL49653 PPARγ ligand (grey) are shown in left Y-axis and the relative induction (x fold induction) due to ligand treatment is shown in right Y-axis (dots).
Figure 3A: Results for control transfection experiment where no PPAR receptor sequence introduced into cells.
Figure 3B: Results for experiments where plasmid (i) contains the wild type PPARγ2 sequence with EGG in first, second, and fifth positions of the P-box. Many of the response elements show activity.
Figure 3C: Results for experiments where plasmid (i) contains a sequence encoding a PPAR polypeptide of the invention [PPARγ2 (GSV)]. The response is specific for the CYP4A6m1 [CYP4A6m-S or SEQ ID No.: 26] sense orientation response element. These results demonstrate the high degree of control possible with the novel PPAR polypeptides. Induction of expression only occurs with the specific combination of PPAR polypeptide and response element selected for use here. These results also demonstrate that the PPAR expression systems of the invention will not cause expression from endogenous response elements, as none of the many wild type response elements have activity with the selected PPAR polypeptide.

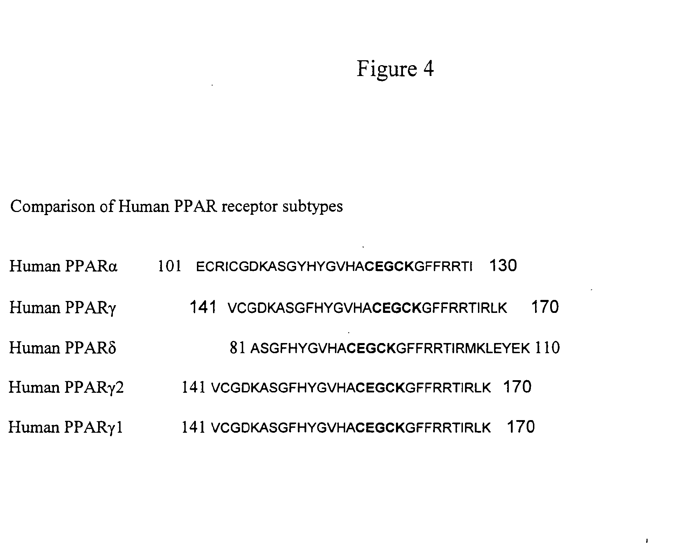

Figure 4: Comparison of a few PPAR receptor site sequences in the regions around the Zinc finger domain. Bolded CEGCKG sequence refers to the sequence indicated in PPAR from Figure 1, panel B, which contains the P-box.

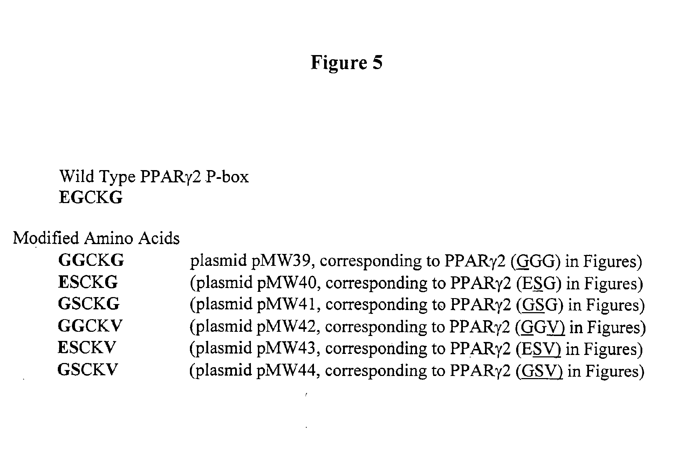

Figure 5: Sequences for the wild type and mutated P-box amino acids in the exemplary PPAR polypeptides tested.
Figure 6: Sequences that can be selected for use as response elements.

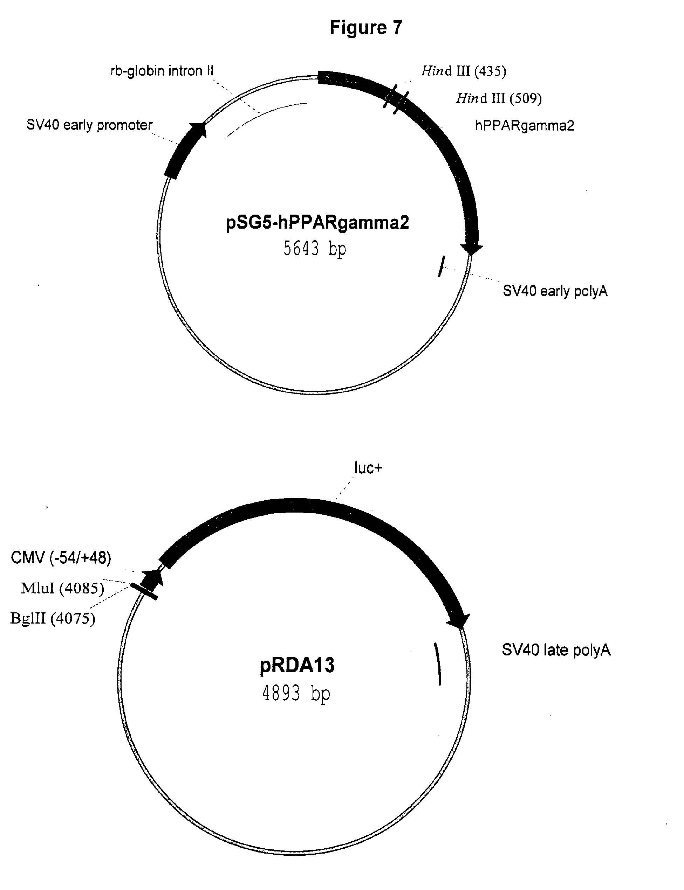

Figure 7: Plasmid maps of pSG5-hPPARgamma2 and pRDA13, noted in the Examples.

Each of the references (publication, article, web page, information source, GenBank or SwissProt sequence, or patent document) referred to in this specification is hereby specifically incorporated herein by reference, in its entirety. Furthermore, each reference or any combination of references can be relied on and used, in whole or in part, to make, use, and test embodiments of the invention or specific examples described here. As this statement applies to each and every reference, document, or source of information, this specification will not repeat the incorporation by reference. This statement operates to effectively incorporate by reference in their entirety each and every reference (as defined above) listed or referred to in the specification.

In making and using aspects and embodiments of this invention, one skilled in the art may employ conventional molecular biology, virology, microbiology, and recombinant DNA techniques. Exemplary techniques are explained fully in the literature and are well known in the art. For example, one may rely on the following general texts to make and use the invention: Sambrook *et al., Molecular Cloning: A Laboratory Manual,* Second Edition (1989) Cold Spring Harbor Laboratory Press, Cold Spring Harbor, New York and Sambrook *et al.* Third Edition (2001); *DNA Cloning: A Practical Approach,* Volumes I and II (D.N. Glover ed. 1985); *Oligonucleotide Synthesis* (M.J. Gaited. 1984); *Nucleic Acid Hybridization* (B.D. Hames & S.J. Higgins eds. (1985)); *Transcription And Translation* Hames & Higgins, eds. (1984); *Animal Cell Culture* (RI. Freshney, ed. (1986)); *Immobilized Cells And Enzymes* (IRL Press, (1986)); B. Perbal, *A Practical Guide To Molecular Cloning* (1984); F.M. Ausubel et al. (eds.), *Current Protocols in Molecular Biology,* John Wiley & Sons, Inc. (2001), Coligan et al. (eds.), *Current Protocols in Immunology,* John Wiley & Sons, Inc. (2001); Dracopdi et al., *Current Protocols in Human Genetics,* John Wiley & Sons, Inc. (2001), W. Paul et al. (eds.) *Fundamental Immunology,* Raven Press; E.J. Murray *et al.* (ed.) *Methods in Molecular Biology: Gene Transfer and Expression Protocols,* The Humana Press Inc. (1991); and J.E. Celis *et al., Cell Biology: A Laboratory Handbook,* Academic Press (1994).

As used herein, a "vector" means any nucleic acid or nucleic acid-bearing particle, cell, or organism capable of being used to transfer a nucleic acid into a host cell. The term "vector" includes both viral and nonviral products and means for introducing the nucleic acid into a cell. Typically, a vector is a recombinant plasmid or animal virus. A "vector" can be used *in vitro, ex vivo,* or *in vivo.* Non-viral vectors include plasmids, cosmids, and can comprise liposomes, electrically charged lipids (cytofectins), DNA-protein complexes, and biopolymers, for example. Viral vectors include retroviruses, lentiviruses, adeno-associated virus, pox viruses, baculovirus, reoviruses, vaccinia viruses, herpes simplex viruses, Epstein-Barr viruses, and adenovirus vectors, for example.

A "nucleic acid" is a polymeric compound comprised of covalently linked nucleotides, from whatever source. Nucleic acid includes polyribonucleic acid (RNA) and polydeoxyribonucleic acid (DNA), both of which may be single-stranded or double-stranded. DNA includes cDNA, genomic DNA, synthetic DNA, and semisynthetic DNA. The term "nucleic acid" also captures sequences that include any of the known base analogues of DNA and RNA.

Percent "identity" between two nucleic acids or two polypeptide molecules refers to the percent defined by a comparison using, for example, a basic blastx, blastn, or blastp search at the default settings (*see, for example,* NCBI BLAST home page: http://www.ncbi.nlm.nih.gov/BLAST/). "Homology" can be determined by a direct comparison of the sequence information between two polypeptide molecules by aligning the sequence information and using readily available computer programs. Alternatively, homology can be determined by hybridization of polynucleotides under conditions allowing for the formation of stable duplexes between homologous regions and determining of identifying double-stranded nucleic acid.

One or more amino acid residues within a sequence can be substituted by another amino acid of a similar polarity, which acts as a functional equivalent when the substitution results in no significant change in activity in at least one selected biological activity or function. A derivative polypeptide will be a functional equivalent of a given amino acid sequence. For example, derivatives of the specific PPAR polypeptides noted or referred to here can be made by incorporating one or more substitutions at selected positions, preferably outside the P-box of the Zinc finger domain. Therefore, various derivatives of the polypeptides of SEQ ID NO: 7 and 8, for example, can be made. Also derivatives can be made by truncations from the C-terminal or N-terminal end of PPAR polypeptides, such as SEQ ID NO: 7 or 8, to produce a functionally equivalent transcriptional transactivator. Conservative substitutions for an amino acid within a sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar, neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Alterations of an amino acid with another amino acid from the same class will not substantially effect activity, apparent molecular weight as determined by polyacrylamide gel electrophoresis, or significantly affect the isoelectric point.

"Isolated," when referring to a nucleic acid, gene, polypeptide, protein, receptor, or vector, means that the indicated molecule is present in the substantial absence of other biological macromolecules of the same type. Thus, an isolated nucleic acid molecule that encodes a particular polypeptide can refer to a nucleic acid molecule substantially free of other nucleic acid molecules that do not encode the particular polypeptide. However, the preparation or sample containing the molecule may include other components of different types. In addition, "isolated from" a particular molecule may also mean that a particular molecule is substantially absent from a preparation or sample.

A "transcription control sequence" means nucleic acid sequences that function to control the rate of operably linked coding regions and/or the fidelity of the transcripts or the polypeptides expressed from them. The phrase includes, for example, promoters, enhancers, poly A sites, and intron sequences comprising splice donor and acceptor sites. One skilled in the art is familiar with and knows how to employ numerous transcriptional control sequences.

As used herein, a "PPAR polypeptide" can refer to a polypeptide capable of binding to a PPRE or a selected response element, or a polypeptide possessing a transcriptional transactivating function directed to a PPRE or selected response element. Various PPAR polypeptides are specifically described in the Examples, Figures, and specific sequences of the Sequence Listing, however many others can be produced from the teachings here. Alternatively, the PPAR polypeptide contains a Zinc finger domain comprising the peptide fragment having amino acids listed in any one of SEQ ID No.: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV]. The novel PPAR polypeptides and nucleic acids specifically those having or encoding sequences that include SEQ ID NO: 7 [PPARγ2 with GSCKG substitutions] and SEQ ID NO: 8 [PPARγ2 with GSCKV substitutions], and any derivative or mutant of SEQ ID NO: 7-8, and any derivative or mutant of a human PPAR receptor that has not previously been disclosed by publication or in an application for patent. The PPAR polypeptide does not include the exact amino acid sequence of SEQ ID NO: 41 [wild type PPARγ2] or the exact sequence of recombinant PPARγ2γ2 of WO 00/7886. As used herein, in addition to the definition given above, a derivative or mutant can be a sequence with about 80% identity, about 90% identity, or about 95% identity with SEQ ID NO: 7 or 8 using blastx, blastp, or blastn at the default settings, as well as nucleotide sequences encoding them, with the proviso that a P-box of the Zinc finger domain that has been modified according to the invention or described in SEQ ID Nos: 1-6 is not changed or is not changed in a manner that substantially alters its ability to bind a specific response element or control transcription from a response element linked to a gene of interest. In one particular type of derivative, the PPAR polypeptides encompass any PPAR with a percentage identity range to a given hPPARγ between about 75 and about 99% identity, such as about 85-95%, about 90-95%, or about 95-99%, can be selected for the amino acids or codons outside the P-box and/or outside the first Zinc finger domain of the PPAR used.

The "response element" refers to a nucleotide sequence that a PPAR polypeptide or wild type PPAR receptor is capable of binding to. In general, the response element is operably linked to a promoter or minimal promoter and a gene of interest so that binding of a PPAR polypeptide causes expression of the gene of interest. A number of response elements are listed in Figure 6. The invention details the process of selecting a particularly preferred response element for a PPAR polypeptide used (*see* Figure 3 data). One or multiple copies of the response element, or even combinations of response elements, can be selected for use in an expression cassette or vector of the invention.

As noted above, the gene of interest can be any protein-encoding, polypeptide-encoding, functional-polypeptide encoding, or transcript-encoding nucleic acid sequence. In the methods for testing a particular PPAR polypeptide with a series of response elements, cells, and ligands, the gene of interest may be a reporter gene, such as luciferase or alkaline phosphatase. In the therapeutic methods of the invention, the gene of interest can be selected from a number of therapeutic proteins or transcripts, or even combinations of therapeutic proteins, combinations of therapeutic transcripts, or combinations of both. In preferred examples, the gene of interest is selected from the class of proteins that are immunomodulatory in mammals, that are anti-angiogenic, that are angiogenic, that are pro-drug converting enzymes or kinases, or that possess tumor-suppressing, anti-proliferative, or anti-tumor characterisitics. Specific, non-limiting examples include genes sequences encoding a particular protein, such as IL-2, IL-12, an interferon, α-interferon, β-interferon, γ-interferon, HSV thymidine kinase (TK) or other TK, Akt-1, Akt-3, or other Akt kinase, GM-CSF, G-CSF, M-CSF, tumor necrosis factor α or β, IL-1, IL-3, IL-4, IL-5, IL-6, IL-11, IL-15, IL-18, angiostatin, endostatin, an amino-terminal fragment of plasminogen having an amino acid sequence of plasminogen from about amino acid residue 1 to about residue 333, anti-angiogenic fragment of plasminogen or angiostatin or endostatin or thrombospondin or platelet factor IV, FGF, aFGF, bFGF, VEGF, or an angiogenic fragment of FGF, aFGF, bFGF, VEGF, a thymidine kinase or cytosine deaminase activity-possessing polypeptide, a serine/threonine kinase Akt activity, Akt-1, Akt-2, Akt-3, p53, Rb, mda-7, rap 1A, DCC, k-rev2, k-rev3, and adenosine deaminase. Additional proteins or fragments can be created, as known in the art. Alternatively, a gene of interest can be a transgene having a sequence that produces a functional transcript, such as with anti-sense sequences like those designed to bind mRNA or DNA in a cell to prevent transcription or translation, a ribozyme, an aptamer, or other active RNA molecules or chimeric nucleic acids.

Numerous gene transfer methods and techniques can be used in conjunction with the invention beyond those specifically described. Many of the references listed can be used in selecting an appropriate gene transfer technique, composition, or delivery method. Gene transfer vectors have been used in a number of mammalian test subjects, including mouse, rabbit, cat, dog, primates, and humans. One of skill in the art is familiar with the techniques and methods appropriate for these mammalian test subjects. *See, for example,* Rosenberg *et al*., *New Eng. J. Med.* 323: 570-78 (1990); Cavazzana-Calvo *et al., Science* 288: 669-72 (2000); Dobson *et al., Brit. Med. J.* 320: 1225 (2000); Buckley *et al., Nature Med.* 6: 623-24 (2000); and the general texts and references listed above.

We have engineered a PPAR system, which in one aspect comprises of two expression cassettes: i) one in which the PPAR polypeptide expression is under the control of a promoter, such as the SV40 promoter, and ii) another in which the expression of a gene of interest is driven by an inducible promoter sequence comprising one or more or preferably multiple response elements responsive to PPAR binding. As a demonstration of some of the advantageous and useful properties of the system and the specific elements of the cassettes, we show the tightly regulated expression of reporter genes in mouse myoblasts. In addition, similar experiments can be performed employing electroporation-enhanced direct transfer of naked plasmid DNA vectors bearing the expression cassettes in conjunction with oral deliver of the induction compound to demonstrate that the PPAR system is functional *in vivo* (*see, for example,* WO 00/78986 or U.S. Prov. Application 60/149,721; or Rivera *et al.,* PNAS 96:8657-62 (1999)). The expression of a gene in mammalian and/or murine skeletal muscle can thus be tightly regulated and effectively controlled in a dose dependent manner.

The invention describes the design of new PPAR mutants, here referred to as "PPAR polypeptides," with unique binding specificity. These PPAR polypeptides can be used in the PPAR system to improve the specificity and safety of expression. Most nuclear receptors, including PPAR and RXR, recognize the hexanucleotide half site AGGTCA, whereas the glucocorticoid receptor (GR) group, which also includes the progesterone (PR), androgen (AR), and mineralocorticoid (MR) receptors, bind to the half site sequence AGAACA. Here, we have used the sequence variation in response elements and DNA-binding Zinc finger domains of the PPAR proteins to construct and determine a method to develop highly specific and tightly regulatable expression systems. A variety of gene transfer and gene therapy-type approaches can employ these systems and the individual elements.

In one example, the wild type PPAR P-box, whose sequence is EGCKG, can be mutated at different positions. A single mutation (**G**GCKG or E**S**CKG), or 2 mutations (**GS**CKG) or 3 mutations (**GS**CK**V**) can be introduced to create a "GR-like" P-box in a new PPAR polypeptide. Of the PPAR isotypes available, three in particular can be used: PPARα, PPARβ [sometimes also called δ], and PPARγ. The recombinant mutant PPARγ2γ2, described in the patent application WO 00/78986, can also be used to create new PPAR polypeptide transactivators with unique binding properties. Any PPAR protein can be selected for use to prepare, test, and use PPAR polypeptides of the invention. Depending on the cell the gene transfer is designed for or the type of therapy contemplated or the ligand desired for the therapy, a particular PPAR isotype may be most appropriate. The natural gamma receptor is involved with increased sensitivity to insulin. Compounds that activate the gamma receptor cause the synthesis of molecules that facilitate insulin action. Thus, gene therapy protocols employing the PPAR expression systems of the invention may be considered to take advantage of an existing insulin-related therapy by using the same PPARγ ligand to treat the insulin-related disease and to induce expression from the PPAR expression system of the invention. Likewise, the PPAR-alpha receptor is involved with lowering triglycerides levels and therapies to combine triglyceride-related disease with gene therapy protocols can be designed. With the knowledge and flexibility the PPAR polypeptides, response elements, and expression systems provided here, one skilled in the art can devise numerous ways to combine traditional drug therapy with gene therapy. One skilled in the art can devise other therapeutic examples where combinations of PPAR polypeptides and PPAR ligands, for example, can be used, including, diabetes, obesity, atherosclerosis, and cancer. *See, for example,* Kersten *et al.,* Nature 405:421-24 (2000). These combinations may allow the treatments (drug and gene therapies) to work together or even against each other.

In a valuable application, the PPAR polypeptide receptors will be used in a PPAR expression system to control expression of transgenes for gene therapy protocols. Because these new receptors will no longer be able to bind endogenous PPREs, or will preferably bind substantially only the exogenous response elements of the expression system, their overexpression and their activation by ligand treatment will not induce the endogenous PPAR-controlled pathways. Therefore, the use of these new PPAR polypeptides will greatly improve the specificity and the safety of the PPAR system in gene therapy protocols and avoid unwanted or deleterious side effects involved in the gene therapy treatment.

The selection of the PPAR ligand or induction compound will, in part, depend on the design of the PPAR polypeptide used and its intended use, as noted above. However, a number of ligands, both natural and synthetic compounds, may be used. Two exemplary classes of compounds, fibrates and thiazolidinediones, are generally known as PPAR ligands and numerous examples of these compounds, and their derivatives and analogues, have been produced, described, and can be selected for use here. The PPARα-activating ligands are, for example, a group of compounds known as fibrates, such as fibric acid and its analogues. Analogues of fibric acid include, for example, gemfibrozyl (Atherosclerosis 114(1) (1995) 61), bezafibrate (Hepatology 21 (1995) 1025), ciprofibrate (BCE&M 9(4) (1995) 825), clofibrate (Drug Safety 11 (1994) 301), fenofibrate (Fenofibrate Monograph, Oxford Clinical Communications, 1995), clinofibrate (Kidney International. 44(6): 1352(1993)), pirinixic acid (Wy-14,643) or 5,8,11,14-eicosatetranoic acid (ETYA). These various compounds are compatible with a biological and/or pharmacological use *in vitro* or *in vivo.*

The PPARγ ligands may also be chosen from natural and synthetic ligands. Natural ligands include fatty acids, prostaglandins, and eicosanoids (for example linoleic acid, linolenic acid, prostaglandin J2, 9-HODE, 5-HODE) and synthetic ligands include the thiazolidinediones, such as in particular rosiglitazone (BRL49653; Avandia), pioglitazone (Actos), troglitazone (Rezulin; see for example Krey G. *et al., Mol. Endocrinol.,* 11: 779-791 (1997); or Kliewer S. and Willson T., *Curr. Opin. in Gen. Dev.* 8: 576-581 (1998)); or compound RG12525), oxyzolinedione (JTT501; WO 00/35437), or rexinoids (LG100268 or LG100754; U.S. Patent 6,214,850).

The doses used may be adapted by persons skilled in the art, on the basis of the *in vivo* data published in the literature. Thus for example, for a form not soluble in water, typical doses of ligand such as BRL49653 are between 5 and 50 mg/kg, for example 30 mg/kg, which make it possible to obtain a plasma concentration close to about 15 µg/ml at least. For a water-soluble form of ligand, whose bioavailability is greater (for example a maleate salt of BRL49653; rosiglitazone maleate), the typical doses are lower, generally less than 5 mg/kg, for example from 0.01 to 1 mg/kg. These doses can be quite obviously adapted by persons skilled in the art as a function of the constructs used, the ligands used, and the desired applications and effects. In general, the results presented in the examples advantageously show that the compositions of the invention make it possible to obtain *in vivo* a high and regulated expression, at ligand doses less than those normally used. In addition, although repeated administrations of ligand may be carried out, a single dose of ligand can also be used.

Generally, the plasmid vector doses used may vary between 0.01 and 1000 µg, or more, depending on the desired applications. The plasmids can be prepared for gene transfer administration by purification with the endo-free Mega-Prep kit (Qiagen). Preferably, the endotoxin level detected in the samples is less than 20 EU per mg of DNA. Other methods and techniques for purifying vectors and nucleic acids for administration to a mammal are known in the art and can be used. In addition, one skilled in the art is familiar with numerous techniques for preparing recombinant viral vectors for use in gene transfer and even therapeutic gene therapy applications.

The invention may be used for expressing a gene in various types of cells, tissues or organs, *in vitro, ex vivo or in vivo.* In particular, this may be a mammalian, preferably a human, cell, tissue or organ. By way of illustration, muscle cells (or a muscle), hepatic cells (or the liver), adipose cells (fat cells), cardiac cells (or the heart, the arterial or vascular wall), nerve cells (or the brain, the marrow and the like) or tumor cells (or a tumor) can be selected. PPAR receptors can be characterized according to their isotype and/or the type of cell they are produced in as a native receptor molecule. *See, for example,* Hseuh, *et al.* Diabetes Care 24: 392-7 (2001); Mochizuki, *et al.,* Arch. Biochem. Biophys. 389: 41-8 (2001); Chinetti, *et al.* Z. Kardiol. 90 (Suppl 3): 125-32 (2001); Ohta, *et al.* J. Clin. Endocrin. Metab. 86: 2170-77 (2001); Desreumaux, *et al.* J. Exp. Med. 193: 827-38 (2001); Mukherjee, *et al.,* J. Biol. Chem. 272: 8071-76 (1997); Murkerjee, *et al.,* J. Steroid Biochem. Molec. Biol. 51: 157-166 (1994); and Yanase, *et al.* Biochem. Biophys. Res. Comm. 233: 320-24 (1997). Any of these sources, cell types, or isotypes of PPAR can be selected as the basis for producing a PPAR polypeptide of the invention or used in selecting elements of an expression system of the invention.

As partial demonstration of the need for and advantages of the PPAR expression system, we assessed the impact of the tetracycline-regulatable, chimeric transcription factor rtTA2M2 (*see, for example,* Urlinger, *et al.,* PNAS 97: 7963-68 (2000)) in gene transfer methods to animals. In this experiment, mice are injected with a plasmid expressing an AP (alkaline phosphatase activity) reporter gene under the control of a constitutive transcription promoter (CMV) or with a plasmid expressing an AP reporter gene under the control of a rtTA2M2-responsive promoter in combination with a rtTA2M2-encoding plasmid. Animals are given doxycycline (a water-soluble derivative of tetracycline) in the drinking water because rtTA2M2 is activated by this molecule. This experiment demonstrates clearly that rtTA2M2 is not capable of sustaining high levels of expression of AP for more than 15 days. This may be due to a cytotoxic immune response against the cells expressing rtTA2M2 and mSEAP (rtTA2M2 is a fusion protein made of domains of bacterial and viral origins). In fact, the data from a related experiment in Latta-Mahieu, *et al.,* Molecular Therapy, vol.3, number 5, part 2, May 2001, abstract 1133, demonstrates that both a cellular and antibody immune response results when primates (cynomolgus macaques) are used as subjects. As the PPAR polypeptides used in the invention are not likely to be seen as foreign to mammals immune response mechanisms, this deleterious immune response can be avoided. The PPAR polypeptide can be selected from the same animals or mammal intended to be used for the gene transfer to further ensure no immune response will result. Thus, the human PPAR receptors can be selected to produce the PPAR polypeptides of the invention when human gene therapy applications are intended. Several of these human PPAR polypeptides are produced and shown below in the Examples.

The results discussed above and in the following specific examples are merely representative of the scope of the invention and content of this disclosure. One skilled in the art can use the information here to devise, produce, and use additional embodiments of the invention. Thus, the examples given here should not be taken as a limitation on the scope or extent of the invention.

### Example 1: Construction of Plasmids Bearing PPAR Polypeptide-Encoding Sequences

The plasmids pSG5 (Stratagene), pBluescript II SK+ (Stratagene) and pSL301 (Invitrogen Corporation) are of commercial origin. The constructions of the expression plasmids pSG5-hPPARγ2 (Fajas L. et al., *J*. *Biol. Chem.,* 272: 18779-18789 (1997)) and pSG5-hPPARα(Koz) (Gervois P. et al., *Mol. Endocrinol.,* 13: 400-409(1999)) have previously been described. Plasmid pSG5-hPPARγ2 (Figure 7) was cut with Hind III and the 74 base-pair fragment encompassing the sequence encoding the P-box was replaced by annealed oligonucleotides of the same sequence except that one, two or three codons for the E, G, G residues of the P-box were mutated into codons for G, S, V residues (plasmids pMW39 to 44, Figure5). The modification of the P-box can also be applied to a recombinant transcriptional regulator comprising two copies of the ligand-binding domain hPPARγ2γ2 (in WO 00/78986 and U.S. Prov. Application 60/149, 721), other recombinant PPAR polypeptides, or any other PPAR receptor or isotype mentioned here or any PPAR protein derived from a cell that produces them.

The P-box amino acids comprise a Zinc finger domain in the PPAR receptor and PPAR polypeptide. By selecting the appropriate oligonucleotides pairs, any of a large number of mutations or substitution changes in the P-box of the Zinc finger domain can be made. According to the methods below, these large numbers of mutant PPAR polypeptides can be tested against a panel of response elements in a particular cell type to determine the optimum combination of PPAR polypeptide, response element, and even PPAR ligand for any particular cell type or group of cell types. Thus, for example, a PPARγ ligand that exerts antiproliferative or apoptotic effects on carcinoma cells can be selected for use. A hPPARγ receptor is used to produce a PPAR polypeptide containing the P-box substitution of SEQ ID No.: 1 or 4, such as in SEQ ID Nos.: 7 and 8. A response element, such as that containing one or more copies of the CYP4A6m1 sequence (SEQ ID No.: 26) can then be selected, as CYP4A6m1 is shown here to be highly selective for a PPAR polypeptide with the GSG or GSV substitutions. The combination of specific antiproliferative PPARγ ligand and PPAR polypeptide may show that another response element, which can be easily identified through the results of Figures 2 and 3, is best for a particular cell type *in vivo* or *in vitro* (i.e. a particular cancer cell). The treatment of the antiproliferative PPARγ ligand and an expression system that uses the same ligand to express a gene of interest, here preferably a gene encoding an anti-tumor, antiproliferative, or immunomodulating function, allows combinatorial therapies for treating diseases such as cancer. Once the cancer treatment is concluded, no further expression of the gene of interest occurs. Furthermore, as a human polypeptide is used in the expression system (the human PPARγ with the P-box mutation), no adverse side effects of the expression system will result. Similarly, treatments that effect vascular cells, cardiac cells, insulin-responsive cells, brain cells, intestinal cells, thyroid cells, muscle cells, liver cells, kidney cells, pancreatic cells, lung cells, testes cells, colon cells, or any other cell can be selected. The PPAR receptors and PPAR receptor isotypes have been defined in a number of different cell types and their ligands connected to a number of different disease treatments. Furthermore, combinations of ligand, PPAR polypeptide, and response element can be selected in an expression system so that no endogenous expression, or substantially no endogenous expression, from the cell's PPAR receptor-based transactivating activity is detected. Figures 2 and 3 show some combinations where substantially no expression occurs from an endogenous or wild type PPRE response element.

The PPAR polypeptides of the invention, and the nucleic acids that encode them, can be prepared from, derived from, or designed using the information of a number of available sources. Some of the sources, in addition to those listed elsewhere in this disclosure, include nucleotide and amino acid sequences from databases. A non-limiting list of some of the sources includes: Biochemistry, 32 (21): 5598-5604 (1993); J. Steroid Biochem. Mol. Biol., 51 (3-4): 157-166 (1994); U.S. Patent 5,686,596; SwissProt accession Q07869; J. Biol. Chem., 272 (12): 8071-8076 (1997); Mol. Cell, 5 (3): 545-555 (2000); Biochem. Biophys. Res. Commun. 241 (2): 270-274 (1997); SwissProt accession P37231; U.S. Patent 5,861,274; Biochem. Biophys. Res. Commun. 224(2): 431-437 (1996); Biochem. Biophys. Res. Commun. 233 (2): 320-324 (1997); SwissProt accession Q03181; Mol. Endocrinol. 6 (10): 1634-1641 (1992); Annu. Rev. Biochem. 70:341-367 (2001); Z Kardiol. 90 Suppl 3:125-32 (2001); Arch Biochem Biophys 389(1): 41-8 (2001). These sources can also be used for designing or selecting response elements, cell types, and ligands for use in the expression systems, or used for any purpose to produce various embodiments of the invention.

### Example 2: Construction of Exemplary Response Elements

Numerous transcriptional control and regulatory elements exist in the art and can be selected for use and analysis. The invention is not limited to the use of any particular element or those specifically exemplified here. The nucleic acid encoding the plasmid pGL3-Basic, used for cloning the various promoter regions, as well as the plasmid pRL-null, are of commercial origin (Promega Corporation) and contain promoters that can be adapted for use. The plasmids encoding the reporter gene luciferase under the control of a minimal human CMV I/E promoter and PPRE were obtained by inserting annealed oligonucleotides encoding 3 PPRE (with a 21 nucleotide distance between PPRE centers) between the BglII and MluI sites of plasmid pRDA13. The consensus PPRE as well as PPRE identified in the promoter of 7 genes were studied: ApoAII, BIF, CYP4A1, CYP4A6, FABP, HMG, MEP (Figure 6). Four versions of each PPRE-containing region were studied: PPRE in sense and anti-sense orientation, and WT or mutant PPRE.

The transcription control sequences used here, a minimal CMV immediate early gene promoter, were selected as sequences that result in low background levels of transcription. Many other minimal promoters can be selected possessing the same characteristics. For example, a minimal human IL-2 promoter sequence or a minimal SV40 promoter. One skilled in the art is familiar with the production and design of minimal promoter sequences and any can be selected for use here. In addition, hGH, bGH, or SV40 late gene poly A sequences, for example, can be selected and inserted 3' to the gene of interest.

The optimization of the number of response elements present in the inducible promoter can be studied in transient transfection assays. As shown previously, the optimum number of response elements in the hPPARγ2γ2 example appears to be between 10 and 15 (WO 00/78986).

### Example 3: Transfection or Infection of Cultured Cells

One skilled in the art is familiar with protocols for transfecting plasmids into cell or infecting cells with virus. As an example, murine myoblasts (C2C12; ATCC: CRL1772) and human HEK293 (ATCC: CRL1573) cells are seeded in 24-well plates (7.5 x 10⁴ cells per well) and grown for 24 h in DMEM supplemented with 10% FCS. Cells are washed in DMEM without serum and transfected in triplicate by adding to the cells 0.5 ml of OptiMEM mixed with various quantities of reporter gene (AP or luc) encoding plasmid, supplemented to 500 ng with a carrier plasmid and LipofectAMINE (2 µl for C2C12, 3 µl for HEK293). Five hours later, the medium containing the DNA and the LipofectAMINE is replaced by 1 ml of DMEM supplemented with FCS (2% for C2C12, 10% for HEK293). Aliquots of the culture medium are collected 2 days post-transfection and can be frozen at -70°C for storage. The cells are rinsed twice with PBS, incubated with 100 µl of 0.2% Triton X-100, 50 mM Tris-HCl pH 7.4, 150 mM NaCl, detached from the plate with a scraper and homogenized by repeated pipetting. The lysate is centrifuged 2 minutes at maximum speed in an eppendorf tabletop centrifuge, and the supernatant stored at -70°C. The results in Figures 2 and 3 show the expression from transfected plamids bearing the luc gene controlled by the binding of various PPAR polypeptides to various response elements.

### Example 4: Intramuscular Gene Transfer

Eight-week-old female Balb/C or C57BL/6 mice (Charles River Laboratories) are anesthetized by intraperitoneal injection of 200 µl ketamine (8.66 mg/ml) mixed with xylazine (0.31 mg/ml) in 150 mM NaCl. The hind legs are shaved. Twenty-five microliters of a nucleic acid vector containing solution in 150 mM NaCl are injected in the tibialis cranialis muscle. Thirty seconds after injection, transcutaneous electric pulses can be applied through stainless steel parallel electrodes connected to an Electro Square Porator, Model T820 (BTX, San Diego, CA), and a TDS 210 oscilloscope (Tektronix, Oregon). If used, the plate electrodes are placed on each side of the leg (plate separation distance 4 mm) and 8 square wave pulses (80 volts, 20 ms each pulse, 1 pulse per second) are applied (Mir *et al.,* 1999).

At different time points relative to DNA injection, 50 µl blood samples are collected from the saphenous vein into heparinized capillary tubes (Hem *et al.,* Lab. Anim. 32:364-68 (1998)). Samples are spun 20 minutes at 2000 rpm in a clinical centrifuge and plasma collected and frozen at -70°C for storage. Conventional alkaline phosphatase or luciferase assays can then be performed to determine the amount of gene expression from a sample.

The results shown in Figures 2 and 3 employ the luciferase reporter gene as the gene of interest. However, numerous other genes can be selected and assayed in like manner by one skilled in the art. In addition, the biological effect of the expressed gene of interest can be determined in animals, including mouse, primate, and human.

The invention described and exemplified above is not limited to the specific embodiments and examples presented here. One skilled in the art can use the techniques and knowledge available through the documents and references noted and specifically incorporated herein, or other documents or references, to make and use additional embodiments. Thus, the description above should not be taken as a limitation of the scope or content of this invention.

## Claims

1. A recombinant vector for expressing a PPAR polypeptide, the vector comprising a nucleic acid encoding the PPAR polypeptide operably linked to transcription control sequences, wherein the PPAR polypeptide comprises a Zinc finger amino acid domain that comprises the amino acids selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV].

2. The vector of claim 1, wherein the Zinc finger amino acid domain comprises SEQ ID No: 1 [GSCKV].

3. The vector of claim 1, wherein the Zinc finger amino acid domain comprises SEQ ID No: 4 [GSCKG].

4. The vector of claim 1, wherein the Zinc finger amino acid domain comprises SEQ ID No: 5 [GGCKV].

5. The vector of claim 1, wherein the PPAR polypeptide comprises SEQ ID No: 8 [PPARγ2 (GSV)].

6. The vector of claim 1, wherein the PPAR polypeptide comprises SEQ ID No: 7 [PPARγ2 (GSG)].

7. The vector of any one of claims 1-6, wherein the transcription control sequences comprise a promoter selected from the group consisting of CMV promoter, SV40 promoter, HSV thymidine kinase promoter, human Apo-AII promoter, and hEF1α promoter.

8. An inducible expression system, comprising:
- a nucleic acid having a sequence comprising an inducible promoter having a response element, the promoter operably linked to a sequence encoding a gene product of interest, wherein activity from the promoter is regulated through the binding of a PPAR polypeptide transactivating factor to the response element; and
- a recombinant vector as claimed in claim 1;
wherein the induction of expression of the gene product of interest is regulated by the presence of the PPAR ligand.

9. The expression system of claim 8, wherein the recombinant vector as claimed in claim 1 comprises a nucleic acid that encodes a Zinc finger amino acid domain comprising SEQ ID No: 1 [GSCKV].

10. The expression system of claim 8, wherein the recombinant vector as claimed in claim 1 comprises a nucleic acid that encodes a Zinc finger amino acid domain comprising SEQ ID No: 4 [GSCKG].

11. The expression system of claim 8, wherein the recombinant vector as claimed in claim 1 comprises a nucleic acid that encodes a Zinc finger amino acid domain comprising SEQ ID: 5 [GGCKV].

12. The expression system of claim 8, wherein the PPAR polypeptide comprises SEQ ID No: 8 [PPARγ2 (GSV)].

13. The expression system of claim 8, wherein the PPAR polypeptide comprises SEQ ID No: 7 [PPARγ2 (GSG)].

14. A recombinant, inducible expression vector comprising a promoter, wherein the promoter is linked to a response element comprising a nucleotide sequence selected from the group consisting of SEQ ID No: 10-40.

15. The recombinant expression vector of claim 14, the response element having a nucleotide sequence of SEQ ID No: 26 [CYP4A6m1].

16. The recombinant expression vector of claim 14, the response element having a nucleotide sequence selected from the group consisting of SEQ ID No: 10, 14, 18, 20, 22, 24, 26, 28, 30, 32, 34, 36, 38, and 40.

17. An inducible expression system, comprising:
- a recombinant vector as claimed in one of claims 14-16, wherein the promoter is operably linked to a nucleic acid encoding a gene product of interest, wherein activity from the promoter is regulated through the binding of a PPAR polypeptide transactivating factor to the response element; and
- a PPAR polypeptide, the PPAR polypeptide comprising a Zinc finger amino acid domain that comprises the amino acids selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV],
wherein the induction of expression of the gene product of interest is regulated by the presence of the PPAR ligand.

18. The expression system of claim 17, the response element having a nucleotide sequence of SEQ ID No: 26 [CYP4A6m1].

19. The expression system of claim 17, the response element having a nucleotide sequence selected from the group consisting of SEQ ID No: 10, 14, 18, 20, 22, 24, 26, 28, 30, 32, 34, 36, 38 and 40.

20. An inducible expression system comprising a recombinant vector, wherein a promoter is operably linked to a nucleic acid encoding a gene product of interest, and wherein activity from the promoter is regulated through the binding of a PPAR polypeptide transactivating factor to a response element, and wherein the PPAR polypeptide comprises a Zinc finger amino acid domain that comprises the sequence of amino acids of SEQ ID No: 1 [GSCKV], the response element having a nucleotide sequence of SEQ ID No: 26 [CYP4A6ml], wherein the induction of expression of the gene product of interest is regulated by the presence of the PPAR ligand.

21. A method of regulating the expression of a gene of interest in a mammalian cell, comprising
introducing into the cell a nucleic acid encoding the PPAR polypeptide operably linked to transcription control sequences, wherein the PPAR polypeptide comprises a Zinc finger amino acid domain that comprises the amino acids selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV];
introducing into the cell a nucleic acid comprising inducible promoter operably linked to a nucleic acid encoding a gene of interest, wherein the promoter is linked to a response element comprising a nucleotide sequence selected from the group consisting of SEQ ID No: 10-40;
and administering a selected PPAR ligand to induce expression of the gene of interest in the cell.

22. The method of claim 21, wherein the cell is a human cell.

23. The method of claim 21, wherein the cell is a primate cell.

24. The method of claim 21, wherein the cell is a mouse cell.

25. The method of claim 21, wherein the cell is a tumor cell.

26. The method of one of claims 21-25, wherein the cell is selected from the group consisting of an adipose cell, a muscle cell, a brain cell, an endothelial cell, a smooth muscle cell, a hepatic cell, a kidney cell, an intestinal cell, or a thyroid cell.

27. A method of screening for a specific response element for the inducible expression of a gene of interest in a mammalian cell, comprising
introducing into the cell a nucleic acid encoding the PPAR polypeptide operably linked to transcription control sequences, wherein the PPAR polypeptide comprises a Zinc finger amino acid domain that comprises the amino acid sequence selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV];
introducing into the cell a nucleic acid comprising inducible promoter operably linked to a nucleic acid encoding a gene of interest, wherein the promoter is linked to a response element;
administering a selected PPAR ligand to induce expression of the gene of interest in the cell;
and detecting the change in the expression level of the gene of interest.

28. The method of claim 27, wherein the cell is a human cell.

29. The method of claim 27, wherein the cell is a primate cell.

30. The method of claim 27, wherein the cell is a mouse cell.

31. The method of claim 27, wherein the cell is a tumor cell.

32. The method of claim 27, wherein the gene of interest encodes a luciferase or alkaline phosphatase activity.

33. The method of one of claims 27-32, wherein the cell is selected from the group consisting of an adipose cell, a muscle cell, a brain cell, an endothelial cell, a smooth muscle cell, a hepatic cell, a kidney cell, an intestinal cell, or a thyroid cell.

34. A polypeptide produced from a vector of one of claims 1-7.

35. A recombinant PPAR polypeptide comprising a Zinc finger amino acid domain that comprises the amino acid sequence selected from the group consisting of SEQ ID No: 1 [GSCKV], SEQ ID No.: 2 [GGCKG], SEQ ID No.: 3 [ESCKG], SEQ ID No.: 4 [GSCKG], SEQ ID No.: 5 [GGCKV], and SEQ ID No.: 6 [ESCKV], or a derivative thereof.

36. A mammalian cell comprising the PPAR polypeptide of claim 35.

37. A human cell comprising the PPAR polypeptide of claim 35.


SEQUENCE LISTING

<110>  Aventis Pharma S.A.

<120>  Inducible Expression Systems Employing PPAR Transcriptional Activators

<130>  GC0026

<160>  41

<170>  PatentIn version 3.1

<210>  1

<211>  5

<212>  PRT

<213>  Artificial Protein

<400>  1

Gly Ser Cys Lys Val
1               5

<210>  2

<211>  5

<212>  PRT

<213>  Artificial

<400>  2

Gly Gly Cys Lys Gly
1               5

<210>  3

<211>  5

<212>  PRT

<213>  Artificial



<400>  3

Glu Ser Cys Lys Gly
1               5


<210>  4

<211>  5

<212>  PRT

<213>  Artificial



<400>  4

Gly Ser Cys Lys Gly
1               5


<210>  5

<211>  5

<212>  PRT

<213>  Artificial



<400>  5

Gly Gly Cys Lys Val
1               5


<210>  6

<211>  5

<212>  PRT

<213>  Artificial



<400>  6

Glu Ser Cys Lys Val
1               5

<210>  7

<211>  505
<212>  DNA

<213>  PPAR(2 recombinant polypeptide comprising GSCKG in Zinc finger domain

<400> 7
Met Gly Glu Thr Leu Gly Asp Ser Pro Ile Asp Pro Glu Ser Asp Ser
1               5                   10                  15

Phe Thr Asp Thr Leu Ser Ala Asn Ile Ser Gln Glu Met Thr Met Val
            20                  25                  30

Asp Thr Glu Met Pro Phe Trp Pro Thr Asn Phe Gly Ile Ser Ser Val
        35                  40                  45

Asp Leu Ser Val Met Glu Asp His Ser His Ser Phe Asp Ile Lys Pro
    50                  55                  60

Phe Thr Thr Val Asp Phe Ser Ser Ile Ser Thr Pro His Tyr Glu Asp
65                  70                  75                  80

Ile Pro Phe Thr Arg Thr Asp Pro Val Val Ala Asp Tyr Lys Tyr Asp
                85                  90                  95

Leu Lys Leu Gln Glu Tyr Gln Ser Ala Ile Lys Val Glu Pro Ala Ser
            100                 105                 110

Pro Pro Tyr Tyr Ser Glu Lys Thr Gln Leu Tyr Asn Lys Pro His Glu
        115                 120                 125

Glu Pro Ser Asn Ser Leu Met Ala Ile Glu Cys Arg Val Cys Gly Asp
    130                 135                 140

Lys Ala Ser Gly Phe His Tyr Gly Val His Ala Cys Gly Ser Cys Lys
145                 150                 155                 160

Gly Phe Phe Arg Arg Thr Ile Arg Leu Lys Leu Ile Tyr Asp Arg Cys
                165                 170                 175

Asp Leu Asn Cys Arg Ile His Lys Lys Ser Arg Asn Lys Cys Gln Tyr
            180                 185                 190

Cys Arg Phe Gln Lys Cys Leu Ala Val Gly Met Ser His Asn Ala Ile
        195                 200                 205

Arg Phe Gly Arg Met Pro Gln Ala Glu Lys Glu Lys Leu Leu Ala Glu
    210                 215                 220

Ile Ser Ser Asp Ile Asp Gln Leu Asn Pro Glu Ser Ala Asp Leu Arg
225                 230                 235                 240

Ala Leu Ala Lys His Leu Tyr Asp Ser Tyr Ile Lys Ser Phe Pro Leu
                245                 250                 255

Thr Lys Ala Lys Ala Arg Ala Ile Leu Thr Gly Lys Thr Thr Asp Lys
            260                 265                 270

Ser Pro Phe Val Ile Tyr Asp Met Asn Ser Leu Met Met Gly Glu Asp
        275                 280                 285

Lys Ile Lys Phe Lys His Ile Thr Pro Leu Gln Glu Gln Ser Lys Glu
    290                 295                 300

Val Ala Ile Arg Ile Phe Gln Gly Cys Gln Phe Arg Ser Val Glu Ala
305                 310                 315                 320

Val Gln Glu Ile Thr Glu Tyr Ala Lys Ser Ile Pro Gly Phe Val Asn
                325                 330                 335

Leu Asp Leu Asn Asp Gln Val Thr Leu Leu Lys Tyr Gly Val His Glu
            340                 345                 350

Ile Ile Tyr Thr Met Leu Ala Ser Leu Met Asn Lys Asp Gly Val Leu
        355                 360                 365

Ile Ser Glu Gly Gln Gly Phe Met Thr Arg Glu Phe Leu Lys Ser Leu
    370                 375                 380

Arg Lys Pro Phe Gly Asp Phe Met Glu Pro Lys Phe Glu Phe Ala Val
385                 390                 395                 400

Lys Phe Asn Ala Leu Glu Leu Asp Asp Ser Asp Leu Ala Ile Phe Ile
                405                 410                 415

Ala Val Ile Ile Leu Ser Gly Asp Arg Pro Gly Leu Leu Asn Val Lys
            420                 425                 430

Pro Ile Glu Asp Ile Gln Asp Asn Leu Leu Gln Ala Leu Glu Leu Gln
        435                 440                 445

Leu Lys Leu Asn His Pro Glu Ser Ser Gln Leu Phe Ala Lys Leu Leu
    450                 455                 460

Gln Lys Met Thr Asp Leu Arg Gln Ile Val Thr Glu His Val Gln Leu
465                 470                 475                 480

Leu Gln Val Ile Lys Lys Thr Glu Thr Asp Met Ser Leu His Pro Leu
                485                 490                 495

Leu Gln Glu Ile Tyr Lys Asp Leu Tyr

<210>  8

<211>  505
<212>  DNA

<213>  PPAR(2 recombinant polypeptide comprising GSCKV in Zinc finger domain

<400> 8
Met Gly Glu Thr Leu Gly Asp Ser Pro Ile Asp Pro Glu Ser Asp Ser
1               5                   10                  15

Phe Thr Asp Thr Leu Ser Ala Asn Ile Ser Gln Glu Met Thr Met Val
            20                  25                  30

Asp Thr Glu Met Pro Phe Trp Pro Thr Asn Phe Gly Ile Ser Ser Val
        35                  40                  45

Asp Leu Ser Val Met Glu Asp His Ser His Ser Phe Asp Ile Lys Pro
50                  55                  60

Phe Thr Thr Val Asp Phe Ser Ser Ile Ser Thr Pro His Tyr Glu Asp
65                  70                  75                  80

Ile Pro Phe Thr Arg Thr Asp Pro Val Val Ala Asp Tyr Lys Tyr Asp
                85                  90                  95

Leu Lys Leu Gln Glu Tyr Gln Ser Ala Ile Lys Val Glu Pro Ala Ser
            100                 105                 110

Pro Pro Tyr Tyr Ser Glu Lys Thr Gln Leu Tyr Asn Lys Pro His Glu
        115                 120                 125

Glu Pro Ser Asn Ser Leu Met Ala Ile Glu Cys Arg Val Cys Gly Asp
    130                 135                 140

Lys Ala Ser Gly Phe His Tyr Gly Val His Ala Cys Gly Ser Cys Lys
145                 150                 155                 160

Val Phe Phe Arg Arg Thr Ile Arg Leu Lys Leu Ile Tyr Asp Arg Cys
                165                 170                 175

Asp Leu Asn Cys Arg Ile His Lys Lys Ser Arg Asn Lys Cys Gln Tyr
            180                 185                 190

Cys Arg Phe Gln Lys Cys Leu Ala Val Gly Met Ser His Asn Ala Ile
        195                 200                 205

Arg Phe Gly Arg Met Pro Gln Ala Glu Lys Glu Lys Leu Leu Ala Glu
    210                 215                 220

Ile Ser Ser Asp Ile Asp Gln Leu Asn Pro Glu Ser Ala Asp Leu Arg
225                 230                 235                 240

Ala Leu Ala Lys His Leu Tyr Asp Ser Tyr Ile Lys Ser Phe Pro Leu
                245                 250                 255

Thr Lys Ala Lys Ala Arg Ala Ile Leu Thr Gly Lys Thr Thr Asp Lys
            260                 265                 270

Ser Pro Phe Val Ile Tyr Asp Met Asn Ser Leu Met Met Gly Glu Asp
        275                 280                 285

Lys Ile Lys Phe Lys His Ile Thr Pro Leu Gln Glu Gln Ser Lys Glu
    290                 295                 300

Val Ala Ile Arg Ile Phe Gln Gly Cys Gln Phe Arg Ser Val Glu Ala
305                 310                 315                 320

Val Gln Glu Ile Thr Glu Tyr Ala Lys Ser Ile Pro Gly Phe Val Asn
                325                 330                 335

Leu Asp Leu Asn Asp Gln Val Thr Leu Leu Lys Tyr Gly Val His Glu
            340                 345                 350

Ile Ile Tyr Thr Met Leu Ala Ser Leu Met Asn Lys Asp Gly Val Leu
        355                 360                 365

Ile Ser Glu Gly Gln Gly Phe Met Thr Arg Glu Phe Leu Lys Ser Leu

370                 375                 380

Arg Lys Pro Phe Gly Asp Phe Met Glu Pro Lys Phe Glu Phe Ala Val
385                 390                 395                 400

Lys Phe Asn Ala Leu Glu Leu Asp Asp Ser Asp Leu Ala Ile Phe Ile
                405                 410                 415

Ala Val Ile Ile Leu Ser Gly Asp Arg Pro Gly Leu Leu Asn Val Lys
            420                 425                 430

Pro Ile Glu Asp Ile Gln Asp Asn Leu Leu Gln Ala Leu Glu Leu Gln
        435                 440                 445

Leu Lys Leu Asn His Pro Glu Ser Ser Gln Leu Phe Ala Lys Leu Leu
    450                 455                 460

Gln Lys Met Thr Asp Leu Arg Gln Ile Val Thr Glu His Val Gln Leu
465                 470                 475                 480

Leu Gln Val Ile Lys Lys Thr Glu Thr Asp Met Ser Leu His Pro Leu
                485                 490                 495

Leu Gln Glu Ile Tyr Lys Asp Leu Tyr

<210>  9

<211>  18

<212>  DNA

<213>  Artificial



<400>  9
aaactaggtc aaaggtca                                                    18


<210>  10

<211>  18

<212>  DNA

<213>  Artificial



<400>  10
aaactagaac aaaggtca                                                    18


<210>  11

<211>  18

<212>  DNA

<213>  Artificial

<400>  11
tgacctatga cctagttt                                                    18

<210>  12

<211>  18

<212>  DNA

<213>  Artificial

<400>  12
tgacctatgt tctagttt                                                    18

<210>  13

<211>  18

<212>  DNA

<213>  Artificial

<400>  13
ctaccagggt aaaggttg                                                    18

<210>  14

<211>  18

<212>  DNA

<213>  Artificial

<400>  14
ctaccagaat aaaggttg                                                    18

<210>  15

<211>  18

<212>  DNA

<213>  Artificial

<400>  15
caacctttac cctggtag                                                    18

<210> 16

<211> 18

<212> DNA

<213> Artificial

<400> 16
caacctttat tctggtag 18

<210> 17

<211> 18

<212> DNA

<213> Artificial

<400> 17
aatgtaggta atagttca 18

<210> 18

<211> 18

<212> DNA

<213> Artificial

<400> 18
aatgtagaaa atagttca 18

<210> 19

<211> 18

<212> DNA

<213> Artificial

<400> 19
tgaactatta cctacatt 18

<210> 20

<211> 18

<212>  DNA

<213>  Artificial

<400>  20
tgaactattt tctacatt                                                    18

<210>  21

<211>  18

<212>  DNA

<213>  Artificial

<400>  21
aaactagggt aaagttca                                                    18

<210>  22

<211>  18

<212>  DNA

<213>  Artificial

<400>  22
aaactagaat aaagttca                                                    18

<210>  23

<211>  18

<212>  DNA

<213>  Artificial

<400>  23
tgaactttac cctagttt                                                    18

<210>  24

<211>  18

<212>  DNA

<213>  Artificial

<400>  24
tgaactttat tctagttt                                                    18

<210>  25

<211>  18

<212>  DNA

<213>  Artificial

<400>  25
gaactagggc aaagttca                                                    18

<210>  26

<211>  18

<212>  DNA

<213>  Artificial

<400>  26
gaactagaac aaagttca                                                    18

<210>  27

<211>  18

<212>  DNA

<213>  Artificial

<400>  27
tgaactttgc cctagttc                                                    18

<210>  28

<211>  18

<212>  DNA

<213>  Artificial

<400>  28
tgaactttgt tctagttc                                                    18

<210>  29

<211>  18

<212>  DNA

<213>  Artificial

<400>  29
aatataggcc ataggtca                                                    18

<210>  30

<211>  18

<212>  DNA

<213>  Artificial

<400>  30
aatatagaac ataggtca                                                    18

<210>  31

<211>  18

<212>  DNA

<213>  Artificial

<400>  31
tgacctatgg cctatatt                                                    18

<210>  32

<211>  18

<212>  DNA

<213>  Artificial

<400>  32
tgacctatgt tctatatt                                                    18

<210>  33

<211>  18

<212>  DNA

<213>  Artificial

<400>  33
aaactgggcc aaaggtct                                                    18

<210>  34

<211>  18

<212>  DNA

<213>  Artificial

<400>  34
aaactggaac aaaggtct                                                    18

<210>  35

<211>  18

<212>  DNA

<213>  Artificial

<400>  35
agacctttgg cccagttt                                                    18

<210>  36

<211>  18

<212>  DNA

<213>  Artificial

<400>  36
agacctttgt tccagttt                                                    18

<210>  37

<211>  18

<212>  DNA

<213>  Artificial

<400>  37
attctgggtc aaagttga                                                    18

<210>  38

<211>  18

<212>  DNA

<213>  Artificial

<400>  38
attctggaac aaagttga                                                    18

<210>  39

<211>  18

<212>  DNA

<213>  Artificial

<400>  39
tcaactttga cccagaat                                                    18

<210>  40

<211>  18

<212>  DNA

<213>  Artificial

<400>  40
tcaactttgt tccagaat                                                    18

<210> 41
<211> 505 amino acids
<212> PRT
<213> Homo sapiens

<400> 41